# EUROPÄISCHE PATENTANMELDUNG

(11) **EP 2 502 556 A1**
(43) Veröffentlichungstag der Anmeldung: **26.09.2012**
(21) Anmeldenummer: 12160736.0
(22) Anmeldetag: 22.03.2012
(51) Int. Cl.: A61B 5/0432

(54) **Elektrokardiogramm**

(30) Priorität: 22.03.2011 DE 102011001482
(71) Anmelder: Nowymed AG, 88682 Salem (DE)
(72) Erfinder: Groz, Peter, 88682 Salem (DE)
(74) Vertreter: Weiss, Peter

(57) **Zusammenfassung**

Elektrokardiogramm (EKG) mit einer Messeinheit (2) und einer Aufzeichnungseinheit (3), wobei die Messeinheit (2) und die Aufzeichnungseinheit (3) voneinander lösbar ausgebildet sind, wobei die Messeinheit (2) und die Aufzeichnungseinheit (3) eine Ableitelektrode (1) umfassen, geeignet zur Aufnahme und/oder Aufzeichnung elektrischer Spannungsänderungen an einem Körper.

## Beschreibung

Die vorliegende Erfindung betrifft ein Elektrokardiogramm nach dem Oberbegriff des Anspruchs 1.

### STAND DER TECHNIK

Elektrokardiogramme sind in unterschiedlichster Art und Weise auf dem Markt erhältlich und bekannt. EKG-Registrierungen werden schon seit Jahrzehnten durchgeführt.

Für Messungen zur Diagnose der elektrischen Spannungsänderungen am Herzen und auf der Körperoberfläche sind im Zeitverlauf viele Verfahren bekannt und Geräte vorhanden. Die Messungen ergeben ein immer wiederkehrendes Bild der Herzaktion. Es wird der Frage nachgegangen, ob körperliche Belastung oder Vorfälle welcher Art auch immer Änderungen in der Herztätigkeit auslösen. Zur Messung der Spannungsänderungen werden an festgelegten Stellen auf der Brust der Patienten Elektroden angebracht, die auf den Rekorder abgeleitet werden, der die Spannungsänderungen zwischen den Elektroden erfasst, messtechnisch verwertet und zur nachfolgenden Auswertung aufzeichnet. Dabei ist es wichtig, dass weder durch die Messung selbst noch durch das Tragen des Gerätes die Herztätigkeit beeinflusst wird.

Die bisherigen Geräte zur Aufzeichnung haben den Nachteil, dass diese im Falle der Erneuerung der Energiequelle oder Übernahme der aufgezeichneten Daten vom Körper des Patienten abgenommen oder über eine elektrisch kontaktierte Kabelverbindung ausgeleitet werden müssen.

Elektrische Kontaktstellen sind erfahrungsgemäß an der feuchten Umgebung störungsanfällig und häufige Ursache von auftretenden Fehlern. Drahtlose Verfahren über Telemetrie benötigen zusätzliche Energie. Diese aus dem Stand der Technik bekannten Lösungen stehen daher im Gegensatz zu einem möglichst kompakten Gerät mit geringem Gewicht.

### AUFGABE DER ERFINDUNG

Es ist daher die Aufgabe der vorliegenden Erfindung, ohne Beeinträchtigung der Anwendungsbedingungen ein Gerätekonzept zu realisieren, das die Gebrauchstauglichkeit verbessert.

### LÖSUNG DER AUFGABE

Die Aufgabe wird durch den kennzeichnenden Teil des Anspruchs 1 gelöst.

Als Elektrokardiogramm wird im Sinne der Erfindung eine Mess- und Aufzeichnungseinheit für die Aufnahme von Herzströmen (EKG) und zu deren Speicherung bezeichnet.

Ein bevorzugtes Ausführungsbeispiel ist ein am Körper des Patienten tragbares Gerät, dass durch seine zweckmäßige Gestaltung eine bessere Gebrauchsfähigkeit als bisher bekannten Systeme gestattet.

Zunächst soll das Gerät in zwei miteinander verbundene Geräteeinheiten aufgeteilt werden. Dies bedeutet im Einzelnen, dass eine Messeinheit und eine Registriereinheit vorhanden ist. Die Messeinheit ist hierbei mit den die elektrischen Spannungsänderungen aufnehmenden Elektroden zur Messung und Aufbereitung der gewonnen Signale verbunden. Diese Signale werden an die unmittelbar daneben angeordnete Aufzeichnungseinheit übertragen.

Da die Messeinheit keinen Energiespeicher enthält, wird vom Energiespeicher der Aufzeichnungseinheit die notwendige Energie kontaktlos induktiv über Wechselstromfelder übertragen. Zur Auswertung der Daten und zur Aufladung der Energiequelle kann die Aufzeichnungseinheit einfach von der Messeinheit abgenommen werden, ohne dass eine elektrische Kontaktverbindung gelöst oder hergestellt werden muss. Die Aufzeichnungseinheit kann kontaktlos mit der Registrier-und Ladeeinheit in Verbindung gebracht werden, die wiederum an einen PC zur Bedienung und Auswertung der auslesbaren Daten angeschlossen werden kann.

Bei doppeltem Vorhandensein der Aufzeichnungseinheiten kann mit der zweiten Aufzeichnungseinheit ohne Zeitverlust die Messung am Patienten fortgesetzt werden. Bei der Erstplatzierung des EKG-Messgerätes am Patienten kann die Aufzeichnungseinheit ebenso einfach von der Messeinheit mit bereits fest platzierten Elektroden abgenommen werden, um die korrekte Funktion der Applizierung am Patienten über die soeben gewonnene Daten zu überprüfen.

Die Geräteeinheiten sind in fest umschlossenen, hermetisch dichten Gehäuseeinheiten untergebracht, so dass ein Wechsel der Energiequelle in der Aufzeichnungseinheit für den Anwender überflüssig ist.

In einem bevorzugten Ausführungsbeispiel dieser Erfindung trägt der zu untersuchende Patient das Messgerät mit beiden Geräteeinheiten mit geeigneten Tragehilfsmitteln wie Gurt oder Halsschlaufe am Körper mit den am Körper fest angelegten Ableitelektroden.

Die Erfindung betrifft eine Mess- und Aufzeichnungseinheit für die Aufnahme von Herzströmen (EKG), zu deren Speicherung das Gerät in zwei voneinander trennbare Geräteeinheiten, Messeinheit und Registriereinheit, aufgeteilt ist. In der Messeinheit befindet sich die Elektronik zur Erfassung der der elektrischen Spannungsänderungen am Herzen mittels daran angebrachter Ableitelektroden. In der Aufzeichnungseinheit werden die kontaktlos übertragenen Signale der Messeinheit dauerhaft aufgezeichnet. Die notwendige elektrische Energie wird außerdem kontaktlos an die Messeinheit übertragen. Die Aufzeichnungseinheit zur kontaktlosen Verbindung mit einer Registrier- und Ladeeinheit zur Auswertung und zum Wiederaufladen von der Messeinheit abgetrennt. Die Aufteilung des EKG in zwei Geräteeinheiten ermöglicht es, die Bedienung und Anwendung wesentlich zu vereinfachen und durch den Verzicht auf elektrische Kontakte zum Auslesen der Daten und Wiederaufladen der Energiequelle eine sichere Bedienung des Gerätes zu gewährleisten.

### FIGURENBESCHREIBUNG

Nachfolgend wird die vorliegende Erfindung beispielhaft an einem bevorzugten Ausführungsbeispiel näher erläutert und beschrieben. Diese zeigen in
Fig. 1 eine erfindungsgemäße Ausführungsform einer Messgeräteausführung am Patienten mit trennbaren Gehäuseeinheiten und fest angebrachten Ableitelektroden am Körper des Patienten;
Fig. 2 die abgenommene Aufzeichnungseinheit, verbunden mit der Registrier- und Ladeeinheit, zur Auslesung der Daten und Aufladen der Energiespeichers.

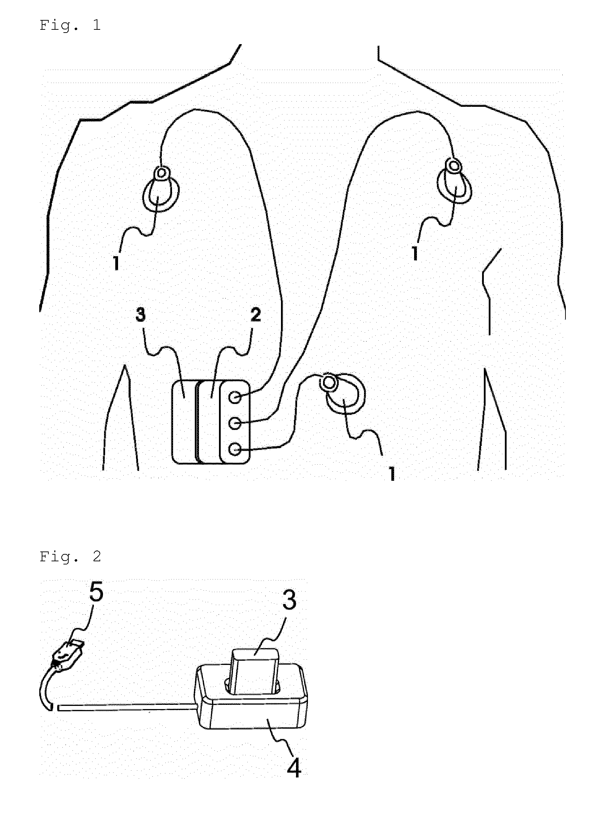

In Fig. 1 werden über definierte Ableitpunkte 1 mittels fest am Körper angebrachten Elektroden die Herzströme abgeleitet und in der Messeinheit 2 messtechnisch erfasst, an der Aufzeichnungseinheit kontaktlos mittels kapazitiven, induktiven, magnetischen Feldänderungen, Funkübertragung, gehäusedurchlässigem Infrarotlicht - oder anderer Lichtübertragungstechnik- übertragen und in der Aufzeichnungseinheit 3 digital aufgezeichnet. Gleichzeitig wird durch Induktion mit Wechselstromfeldern die notwendige Energie an die Messeinheit kontaktlos übertragen. Die Messeinheit benötigt daher keinen Energieträger.

In Fig. 2 ist die Aufzeichnungseinheit 3 von der Messeinheit getrennt und kontaktlos mit der Registrier-und Ladeeinheit 4 in Verbindung gebracht. Dargestellt ist die Phase zur Datenübertragung und Aufladung des Energiespeichers der Aufzeichnungseinheit 3 mittels der Registrier- und Ladeeinheit 4, die mit dem Kabel 5 mit einem Auswertegerät PC verbunden werden kann.

### Positionszahlenliste

1 - Ableitpunkt
2 - Messeinheit
3 - Aufzeichnungseinheit
4 - Ladeeinheit
5 - Kabel
PC - Auswertegerät

## Patentansprüche

1. Elektrokardiogramm (EKG) mit einer Messeinheit (2) und einer Aufzeichnungseinheit (3), wobei die Messeinheit (2) und die Aufzeichnungseinheit (3) voneinander lösbar ausgebildet sind,
**dadurch gekennzeichnet,**
**dass** die Messeinheit (2) und die Aufzeichnungseinheit (3) eine Ableitelektrode (3) umfassen, geeignet zur Aufnahme und/oder Aufzeichnung elektrischer Spannungsänderungen an einem Körper.

2. Elektrokardiogramm (EKG) nach Anspruch 1, **dadurch gekennzeichnet, dass** elektrische Energie kontaktlos über Wechselstromfelder von der Aufzeichnungseinheit (3) zur Messeinheit (2) übertragbar ist.

3. Elektrokardiogramm (EKG) nach Anspruch 1 oder 2, **dadurch gekennzeichnet, dass** die Messeinheit (2) an die direkt angebrachte Aufzeichnungseinheit (3) die aufgenommenen Messdaten ohne elektrische Kontakte mittels kapazitiven, induktiven, magnetischen Feldänderungen, Funkübertragung, gehäusedurchlässigem Infrarotlicht - oder anderer Lichtübertragungstechnik-überträgt.

4. Elektrokardiogramm (EKG) nach einem der vorigen Ansprüche, **dadurch gekennzeichnet, dass** die Aufzeichnungseinheit (3) zur Überprüfung der EKG-Funktion, zur Übernahme der Daten und zur Wiederaufladung des Energiespeichers von der Messeinheit (3) abtrennbar ist, wobei die Aufzeichnungseinheit (2) kontaktlos mit einer Registrier- und Ladeeinheit (4) in Verbindung bringbar ist.

5. Elektrokardiogramm (EKG) nach Anspruch 4, **dadurch gekennzeichnet, dass** die Aufzeichnungseinheit (2) gespeichert Daten und die Neuinitialisierung mittels kapazitiven, induktiven, magnetischen Feldänderungen, Funkübertragung, gehäusedurchlässigem Infrarotlicht - oder anderer Lichtübertragungstechnik- mit der Registrier- und Ladeeinheit (4) austauscht.
